# EUROPEAN PATENT APPLICATION

(11) **EP 3 248 592 A1**
(43) Date of publication of application: **29.11.2017**
(21) Application number: 16171320.1
(22) Date of filing: 25.05.2016
(51) Int. Cl.: A61K 9/00, A61K 9/24, A61K 9/28, A61K 31/568, A61K 31/506, A61K 31/519

(54) **PROCESS FOR THE PREPARATION OF DRUG DELIVERY SYSTEMS HAVING A TESTOSTERONE COMPOUND PRESENT IN AN OUTER LAYER OR PART, AS WELL AS SUCH DRUG DELIVERY SYSTEMS**

(71) Applicant: EB IP Hybritabs B.V., 1311 RL Almere (NL)
(72) Inventor: DE LEEDE, Leonardus Gerardus Jozef, 1311 RL Almere (NL); FRIJLINK, Henderik Willem, 1311 RL Almere (NL); EISSENS, Anko Cornelus, 1311 RL Almere (NL); WILLIAMS, Mark, 1311 RL Almere (NL); TUITEN, Jan Johan Adriaan, 1311 RL Almere (NL)
(74) Representative: V.O.

(57) **Abstract**

The invention relates to a process for preparing a time controlled, immediate release drug delivery system for oral administration of a first active ingredient to a subject in need thereof. The invention additionally relates to a dual drug delivery device, comprising the time controlled, immediate release drug delivery system according to the invention, further comprising a spray coating comprising a testosterone.

## Description

The present invention relates to the field of drug formulation and drug delivery. More specifically, the invention relates to a time controlled, immediate release drug delivery system. The invention additionally relates to a dual drug delivery device comprising the time controlled, immediate release drug delivery system for time controlled, immediate release of a first active ingredient and the preceding release of a testosterone (or a testosterone analogue or derivative), preferably in the mouth, and more preferably sublingual administration of the testosterone. Even more specifically, the invention relates to a combination therapy for the treatment of male or female sexual dysfunction, such as decreased/absence of sexual desire, sexual arousal problems, or erectile dysfunction. Preferably, said combination treatment is for the treatment of Hypoactive Sexual Desire Disorder (HSDD) in men, and/or Female Sexual Interest / Arousal Disorder (FSIAD). Actually before 2013, those skilled in the art referred to HSDD in both men and women; yet in 2013 a new diagnostic classification was adopted. Preferably, a combination of testosterone or a functional analogue or derivative thereof and a first active ingredient is used, whereby the testosterone or a functional analogue or derivative thereof is provided such that the peak plasma level of testosterone occurs about 2-6 hours, more preferred 3-4 hours, prior to the peak plasma level of the first active ingredient.

Such drug delivery systems are described in WO 2012/158030. The present invention forms an improvement on specific embodiments described therein.

Dual drug delivery devices are designed to release a drug at 2 different rates or in 2 different periods of time, or to release two or more different drugs at different periods of time in different compartments. Dual drug delivery devices control the release rate of one or more drugs to maximize the therapeutic effect of these drugs. In a first phase, the drug is quickly released to provide maximum relief or effect within a short time frame. This is followed by a second release phase to avoid a need for repeated frequent administration.

Suitable devices for use as a biphasic release system are compressed double-layer tablets and "core-within-coating" systems, which involves the use of a controlled release tablet as a compressed core which is coated over the whole surface with a disintegrating formulation. Both the core tablet and the outer coating contain a drug.

The present invention is directed to a drug delivery system that releases a drug after a predetermined period of time (a lag time) following administration of the drug delivery system.

The first active ingredient that is released after some time is an active ingredient for treatment male or female sexual dysfunction, such as decreased/absence of sexual desire, sexual arousal problems, or erectile dysfunction, and preferably for the treatment of Hypoactive Sexual Desire Disorder in men, or Female Sexual Interest / Arousal Disorder, is selected from the group consisting of a PDE5 inhibitor, an inhibitor of neutral endopeptidase (NEP) and a 5-hydroxytryptamine 1A receptor agonist (5-HT1Ara). A PDE5 inhibitor is preferably chosen from vardenafil, sildenafil and tadalafil or any of the other known PDE5-inhibitors. Further non-limiting examples of PDE5 inhibitors are: E-4021, E-8010, E-4010, AWD-12-217 (zaprinast), AWD-12-210, UK-343,664, UK-369003, UK-357903, BMS-341400, BMS-223131, FR226807, FR- 229934, EMR-6203, Sch-51866, IC485, TA-1790 (avanafil), DA-8159 (udenafil), NCX-911 or KS-505a. Other examples can be found in WO 96/26940. A typical example for oral administration of vardenafil is provided by vardenafil HCl which is designated chemically as piperazine,1-[[3-(1,4-dihydro-5-methyl-4-oxo-7-propylimidazo[5,1-/] [1,2,4]triazin-2-yl)-4-ethoxyphenyl]sulfonyl]-4-ethyl-monohydrochloride. An other example is given in sildenafil citrate which is chemically designated as 1-[[3-(6,7-dihydro-1-methyl-7-oxo-3-propyl-1H-pyrazolo[4,3-cr]pyrimidin-5-yl)-4-ethoxyphenyl]sulfonyl]-4-methylpiperazine citrate.

A preferred PDE5-inhibitor according to the invention is sildenafil which is preferably administered as sildenafil citrate (1-[[3-(6,7-dihydro-1-methyl-7-oxo-3-propyl-1H-pyrazolo[4,3-cr]pyrimidin-5-yl)-4-ethoxyphenyl]sulfonyl]-4-methylpiperazine citrate).

A further preferred first active ingredient for the treatment of male or female sexual dysfunction, decreased/absence of sexual desire, sexual arousal problems, or erectile dysfunction, and preferably for the treatment of Hypoactive Sexual Desire Disorder in men, or Female Sexual Interest / Arousal Disorder, is an inhibitor of neutral endopeptidase (NEP).

A preferred NEP-inhibitor is selected from candoxatril; candoxatrilat; dexecadotril ((+)-N-[2(R)-(acetylthiomethyl)-3-phenylpropionyl]glycine benzyl ester); CGS-24128 (3-[3-(biphenyl-4-yl)-2-(phosphonomethylamino)-propionamido]propionic acid); CGS-24592 ((S)-3-[3-(biphenyl-4-yl)-2-(phosphonomethylamino)propionamido]propionic acid); CGS-25155 (N-[9(R)-(acetylthiomethyl)-10-oxo-1-azacyclodecan-2(S)-ylcarbonyl]-4(R)-hydroxy-L-proline benzyl ester); 3-(1-carbamoylcyclohexyl)propionic acid derivatives described in WO 2006/027680; JMV-390-1 (2(R)-benzyl-3-(N-hydroxy-carbamoyl)propionyl-L-isoleucyl-L-leucine); ecadotril; phosphoramidon; retrothiorphan; RU-42827 (2-(mercaptomethyl)-N-(4-pyridinyl) benzenepropionamide); RU-44004 (N-(4-morpholinyl)-3-phenyl-2-(sulfanylmethyl)propionamide); SCH-32615 ((S)-N-[N-(1-carboxy-2-phenylethyl)-L-phenylalanyl]-(3-alanine) and its prodrug SCH-34826 ((S)-N-[N-[1-[[(2,2-dimethyl-1,3-dioxolan-4-yl)methoxy]carbonyl]-2-phenylethyl]-L-phenylalanyl]-(3-alanine); sialorphin; SCH-42495 (N-[2(S)-(acetylsulfanylmethyl) - 3- (2 - methylphenyl)propionyl] - L- methionine ethyl ester); spinorphin; SQ-28132 (N-[2-(mercaptomethyl)-1-oxo-3-phenylpropyl]leucine); SQ-28603 (N-[2-(mercaptomethyl)-1-oxo-3-phenylpropyl]-(3-alanine); SQ-29072 (7-[[2-(mercaptomethyl)-1-oxo-3-phenylpropyl]amino]heptanoic acid); thiorphan and its prodrug racecadotril; UK-69578 (cis-4-[[[1-[2-carboxy-3-(2-methoxyethoxy)propyl] cyclopentyl] carbonyl] amino] cyclohexanecarboxylic acid); UK-447,841 (2-{1-[3-(4-chlorophenyl)propylcarbamoyl]-cyclopentylmethyl}-4-methoxybutyric acid); UK-505,749 ((R)-2-methyl-3-{1-[3-(2-methylbenzothiazol-6-yl)propylcarbamoyl]cyclopentyl}propionic acid); 5-biphenyl-4-yl-4-(3-carboxypropionylamino)-2-methylpentanoic acid and 5-biphenyl-4-yl-4-(3-carboxypropionylamino)-2-methylpentanoic acid ethyl ester (WO 2007/056546); daglutril [(3S,2'R)-3-{1-[2'-(ethoxycarbonyl)-4'-phenylbutyl]-cyclopentan-1-carbonylamino}-2,3,4,5-tetrahydro-2-oxo-1H-1-benzazepine-1-acetic acid] described in WO 2007/106708; and combinations thereof.

A preferred NEP inhibitor according to the invention is selective for NEP (EC 3.4. 24.11) over soluble secreted endopeptidase (SEP). NEP degrades a hormone called vasoactive intestinal peptide (VIP) that promotes blood flow to the vagina. Neuropeptides such as vasoactive intestinal peptide (VIP) are major neurotransmitters in the control of genital blood flow. VIP and other neuropeptides are degraded/metabolised by NEP. Thus, NEP inhibitors will potentiate the endogenous vasorelaxant effect of VIP released during arousal. This will lead to enhanced genital blood flow and hence genital engorgement. Selective inhibitors of NEP enhance pelvic nerve-stimulated and VIP-induced increases in vaginal and clitoral blood flow. In addition, selective NEP inhibitors enhance VIP and nerve-mediated relaxations of isolated vagina wall. Therefore, the effects of a NEP-inhibitor are similar to the effects of a PDE5-inhibitor, namely increased vaginal and clitoral blood flow. Preferred NEP inhibitors are UK-447,841 and UK-505,749.

A further preferred first active ingredient preferably for treatment of male or female sexual dysfunction, decreased/absence of sexual desire, sexual arousal problems, or erectile dysfunction, and preferably for the treatment of Hypoactive Sexual Desire Disorder in men, or Female Sexual Interest / Arousal Disorder, is a 5-hydroxytryptamine 1A receptor agonist (5-HT1Ara). Preferably, a 5-HT1Ara is selective for the 5-HT1A receptor over other 5-HT receptors and the α-adrenoreceptor and dopamine receptor. Non-limiting examples of a 5-HT1Ara are 8-OH-DPAT, Alnespirone, AP-521, Buspar, Buspirone, Dipropyl-5-CT, DU-125530, E6265, Ebalzotan, Eptapirone, Flesinoxan, Flibanserin, Gepirone, Ipsapirone, Lesopitron, LY293284, LY301317, MKC242, R(+)-UH-301, Repinotan, SR57746A, Sunepitron, SUN-N4057, Tandosporine, U-92016A, Urapidil, VML- 670, Zalospirone and Zaprasidone. A preferred 5HT1A receptor agonist is buspirone, and more specifically buspirone hydrochloride (HCl).

It is further preferred that a first active ingredient in a time controlled, immediate release drug delivery system according to the invention is a combination of two or more active ingredients such as, but not limited to, two or more PDE5 inhibitors, two or more NEP inhibitors, two or more 5-HT1A receptor agonists, or a combination of at least one PDE5 inhibitor and at least one NEP inhibitor, a combination of at least one PDE5 inhibitor and at least one 5-HT1A receptor agonist, a combination of at least one NEP inhibitor and at least one 5-HT1A receptor agonist, and a combination of at least one PDE5 inhibitor, at least one NEP inhibitor and at least one 5-HT1A receptor agonist.

This first active ingredient is present in a time controlled, immediate release drug delivery device as described in WO 2012/158030, which is surrounded by a layer or other coating comprising testosterone or a functional analogue thereof.

Preferably, the testosterone or a functional analogue or derivative is provided in an immediate release formulation and the first active ingredient is provided in the core of a time controlled, immediate release drug delivery system.

The term "testosterone" *per se,* but also the term "testosterone or (functional) analogue or derivative thereof", as used in the present description and the appending claims, refer to testosterone or a precursor or metabolite of testosterone that provides the same or a similar function as testosterone. Preferred precursors of testosterone are selected from pregnenolone, 17α-hydroxypregnenolone, progesterone, 17α-hydroxy-progesterone, dehydroepiandrosterone, androstenedione, and androstenediol. Preferred metabolites of testosterone are selected from hydroxyandrostene-dione, hydroxytestosterone, including 2β -, 6β -, 7α-, 12α-, and 16α-hydroxytestosterone, and dihydrotestosterone, including 5 α- and 5β-dihydrotestosterone. A preferred analogue of testosterone is capable of binding to an androgen receptor. It is most preferred that said testosterone or a functional analogue thereof is testosterone.

Said "testosterone or functional analogue or derivative thereof" in the second part is preferably combined with a PDE5-inhibitor, a NEP-inhibitor, and/or a 5-HT1A receptor agonist. A dual drug delivery device, comprising a time controlled, immediate release drug delivery system comprising a PDE5-inhibitor, a NEP-inhibitor, and/or a 5-HT1A receptor agonist according the invention, wherein the first coating of the drug delivery system is surrounded by a spray coating comprising testosterone or functional analogue thereof preferably provides the provision of the drug delivery system comprising a PDE5-inhibitor, a NEP-inhibitor, and/or a 5-HT1A receptor agonist between 1.5-5 hours, more preferred 2-3 hours, more preferred about 2.5 hours, after the provision of testosterone or functional analogue thereof.

Another example of a second active agent in a dual drug delivery device according to the invention is provided by estradiol or an analogue or derivative thereof for the treatment of osteoporosis. Said estradiol or analogues thereof may be provided with one or more of an additional drug that is used in the treatment of osteoporosis as a first active agent. An example of said additional drug is a calcium regulator such as alendronate, clodronate, etidronate, pamidronate, risedronate, tiludronate and/or ibandronate; a calcium salt such as, for example, calcium phosphate and/or calcium carbonate; and/or a vitamin D derivative such as, for example, cholecalciferol, calcitriol and/or alfa-calcidol. Said estradiol or analogue or derivative thereof may be replaced as a second active agent by a selective estrogen receptor modulator (SERM), for example Raloxifene, or by parathyroid hormone, for example recombinant parathyroid hormone such as teriparatide. SERM and parathyroid hormone may also be provided with one or more of an additional drug that is used in the treatment of osteoporosis as a first active agent, as is indicated hereinabove.

A further example of a second active agent in a dual drug delivery device according to the invention is provided by nitroglycerin for the treament of angina pectoris. Oral, for example sublingual, dosing of nitroglycerin is preferably combined with a time controlled, immediate release drug delivery system comprising one or more of an additional angina drug as a first active agent. Said additional angina drug is preferably a beta-blocker such as, for example, atenolol, pindolol, propranolol, oxprenolol, metoprolol and/or bisoprolol; a calcium antagonist such as, for example, amlodipine, diltiazem, nifedipine, bepridil, barnidipine, nicardipine and verapamil; and/or a selective heart-rate reducing agent such as, for example, ivabradine.

As mentioned herein-above, delivery systems of the type to which the present invention relates encompass compressed double-layer tablets and "core-within-coating" systems. The present inventors in trying to prepare suitable systems were confronted with the problem that especially the testosterone that needs to be quickly released, already in the mouth, and preferably sublingually, is not delivered immediately in a reliable and consistent amount. That is, it appeared difficult to find an industrial method to provide an outer layer or outer part on a core comprising the first active ingredient, which outer layer or outer part contains a predetermined amount of testosterone, wherein the spreading in the amount of testosterone when determined for a high number of tablets or other system units is very small and according to well-defined pharmaceutical standards.

When using press coating, for example, it was found impossible to create a system, wherein the testosterone is already taken up in the mouth in the desired amount. Press coating requires quite some material to be effectively pressed on a core comprising the first ingredient. Not only gives this larger drug delivery systems providing disadvantages for a person using these systems, but also this results in either a powdery taste and texture in the mouth and/or an insufficient amount of testosterone released in and taken up by the mouth to the systemic circulation.

The use of a quickly disintegrating coating mixture for the press coat material may reduce these disadvantages, but does not solve these.

In two of the working examples of WO 2012/158030, on a laboratory scale tablets were coated using a spray coating composition using one spray nozzle and a small glass spraying-vessel or perforated drum film coater. In such lab scale experiments, it is easy to steer the amounts of testosterone in a spray coat.

When attempting to scale up this process, the present inventors encountered however the problem of getting unreliable testosterone amounts in the final tablets after the spray coating step.

The present inventors have now found an industrial scale process for providing a drug delivery device that combines a drug delivery system that is effective in delivering the first active ingredient in a short pulse after a predetermined period of time with a drug delivery system that provides immediate and reliable (in amount) release of testosterone or an analogue thereof at an earlier point in time after administration, preferably in the oral cavity.

This process provides drug delivery devices or systems of the type described in WO 2012/158030.

In the first aspect, the invention relates to an industrial process for preparing dual drug delivery devices for oral administration of a second and first active ingredient to a subject in need thereof, said process comprising the steps of (i) providing a time controlled, immediate release drug delivery system comprising a core comprising cellulose, a filler selected from an organic and/or an inorganic salt, and the first active ingredient, being an active ingredient for treatment male or female sexual dysfunction, such as decreased/absence of sexual desire, sexual arousal problems, or erectile dysfunction, and preferably for the treatment of Hypoactive Sexual Desire Disorder in men, or Female Sexual Interest / Arousal Disorder, and a first coating surrounding said core, said first coating comprising a hydrophobic polymer and a hydrophilic substance; and (ii) spray coating said time controlled, immediate release drug delivery systems with testosterone or a functional analogue thereof in amounts within 10% and preferably 7,5%, more preferably within 5%, most preferably within 4% of the desired pharmaceutical amounts.

The term "industrial process" means that considerably more devices, such as tablets, are prepared than on the laboratory scale as described in WO 2012/158030. That is, the spray coating process should be carried out such that amounts of more than, say, 2500 tablets are prepared in a reliable way.

In line with a preferred embodiment of said WO 2012/158030, the first coating referred to in step (i) is also applied using the technique of spray coating. When herein-below, reference is made to a "first spray coating", then this is a spray coating in step (i). If alternatively the spray coating of step (ii) is intended, reference is either made to "second spray coating" or "testosterone spray coating".

One important finding reported in Fig 5 and Example 6 of WO 2012/158030 was that it is important for determining the end point for the coating process with the testosterone coat to use the weight of the coating solution sprayed as indicator and not the weight gain of the tablets.

This steering mechanism is also used in the present invention.

In the testosterone spray coating step, the testosterone or its functional analogue is present in a solution containing at least one film forming polymer. Film forming polymers suitable for spray coating are well-known to the skilled person active in this field and may for example be hydroxypropylmethylcellulose, polyvinylpyrrolidone or povidone, hydroxyethylcellulose, other modified celluloses known in the art, polyacrylates, polymethacrylates, and polymethyl/ethylmethacrylates. In tests, the inventors found that the use of polyvinylpyrrolidone resulted in higher moisture sorption of the final pharmaceutical system, making it less preferred than for example hydroxypropylmethylcellulose. A film forming ingredient according to the invention preferably comprises hydroxypropylmethylcellulose, more preferred low molecular weight hydroxypropylmethylcellulose with a number average molecular weight below 20,000; more preferred below 10,000.

As solvent for the testosterone spray coating solution, the present invention requires, or at least preferably uses, a mixture of ethanol and water; preferably a mixture of 60-80 wt.% ethanol and 40-20 wt.% water, such as a mixture of 70 wt.% ethanol and 30 wt.% water.

In a preferred embodiment, the spray coating solution used further comprises a solubilizer, such as polyvinylpyrrolidone (PVP) and preferably a cyclodextrin, or a derivative or polymer thereof. Very good results are obtained using hydroxypropyl ß-cyclodextrin. The amount of solubilizer is preferably in the range of 0.5-3 relative to the weight amount of film forming polymer. Cyclodextrin not only works as a solubilizer, but also avoids or reduces the crystallization of testosterone during storage.

In a further preferred embodiment, the testosterone spray coating solution comprises a flavouring compound and/or a sweetener. Especially, embodiments wherein a flavor is introduced in said spray coating layer in an amount allowing steering as to how long the device is to be held in the mouth to facilitate uptake of the testosterone or its functional analogue in the mouth are preferred. Suitable flavouring compounds and sweeteners encompass peppermint oil, menthol and aspartame.

When trying to scale-up the process described in WO 2012/158030 and go from a small laboratory-scale manufacturing process to larger production-scale processes, the inventors found - with an eye on reliable amounts of testosterone or its functional analogue in the spray coating layer - that the temperature used for drying the tablets, said temperature being 60-80°C in the process used in WO 2012/158030 resulted in uncontrolled amounts of testosterone in the spray coating layers of the individual tablets.

In a preferred embodiment of the process of the present invention, the testosterone spray coating step (ii) inclusive of its inherent drying step is carried out at temperatures up to maximally 55°C, preferably up to maximally 50°C, and more preferably at temperatures in the range of 35°C to 45°C.

In this embodiment the temperature of the supply air is typically 50-60°C.

Preferably, the spray coating steps whether in step (i) or in step (ii) are carried out using a perforated pan coater or fluid bed coater, using tablet bed or exhaust air temperatures in the ranges mentioned in the previous paragraph. To make batch sizes of more than 7 kg, a coating pan is used having a diameter of about 50 cm. This coating pan is rotated with a pan speed of about 14-15 rpm for the first coating and about 19 rpm for the second coating. As suitable coating nozzle, a 1.2 mm nozzle is used; for example a Schlick model 930/7-1 S35 (ex Düsen-Schlick GmbH, Germany). In such a coating pan, the coating time of the first spray coating is about 4 hours or more, using an air pressure (air temperature about 50-60°C) sufficient to form an visibly fine mist during spraying and so that the tablets will have a tablet temperature and exhaust air temperature of about 40°C. The applied target spray rate in this first spray coating step is 35 - 40 g/min and the atomizing air pressure around 1,0 bar and pattern air pressure around 1,4 bar.

Just like in WO 2012/158030, the first coating around the core of the system in step i) comprises preferably a cellulose derivative and especially ethylcellulose as hydrophobic polymer.

Especially in this preferred embodiment, the used maximum temperatures in the spray coating step results in devices, such as tablets, which were found not to be entirely storage stable. More in particular, it was found that the dissolution characteristics of the first active ingredient, such as Sildenafil or Buspirone, were delayed after storage under both long-term and accelerated conditions. It became apparent that the nature of the ethylcellulose polymer coating changed during storage, which, in turn, changed its swelling properties over time. It is hypothesized that this is due to the rearrangement of the polymer chains of the preferred cellulose derivative and especially the ethylcellulose polymer present in the first coating around the first active ingredient of the devices or tablets. In other words, during storage, these polymer chains cure. This curing reduces the speed of water uptake.

To overcome this problem, it is possible to store the tablets in a refrigerator, but that is not practical; or to add excipients with plasticizing properties, but these will likely influence the desired release profile.

The present inventors have solved this instability problem by applying the first coating around the core of the provided system i) at a temperature of 60°C or higher.

Alternatively and preferably, the provided time controlled, immediate release drug delivery system of step i) is subjected to a curing step. This curing step may encompass an additional heating step after application of the first coating layer, the barrier coating. This may be achieved in for instance the spray coating container before spraying or in a fluid bed or in an oven after the application of the barrier coating.

The first coating layer may be formulated to give a short sustained release by forming a coat that slowly dissolves or to give an immediate release by forming a coat that dissolves quickly. The amount of a film-forming ingredient is preferably between 0.05 and 40 % (w/w), based on the total weight of the spray coating, more preferred between 1 and 30 % (w/w) such as, for example, about 20% (w/w).

The second spray coating (layer) preferably comprises a weight of between 0.5 and 5% (w/w) based on the total weight of the drug delivery device. Preferably said coating comprises a weight of between 1% and 3% and preferably between 1,5 and 2,5 % (w/w) based on the total weight of the drug delivery device. In a preferred embodiment the second spray coating of a drug delivery system comprises a weight of between about 1-20 mg per unit. Preferably said spray coating comprises a weight of about 3-15 mg per unit. In a particularly preferred embodiment said spray coating of a drug delivery device of the invention comprises a weight of about 4-10 mg per unit.

The amount of testosterone or its functional analogue is preferably between 0.05 and 25 % (w/w), based on the total weight of the second spray coating layer, more preferred between 0.5 and 15 % (w/w). In amounts it is preferably present in amounts between 0.1-1.5 mg/device.

The spray coating layer of the dual drug delivery device according to the invention preferably provides immediate delivery of the testosterone in the mouth. The term "mouth" comprises the interspace between the lips and the teeth, the interspace between the cheek and the teeth, the oral cavity which is delimited by the palate and tongue and the sublingual area. The testosterone is preferably released in the sublingual space in the mouth.

The phrase "immediate release of the testosterone" refers to the rapid dissolution of the spray coating in the mouth such that the testosterone is completely or substantially completely released within a short time frame within the mouth. The phrase "immediate release of the second ingredient" indicates that at least 50% of the testosterone is released within 5 minutes, more preferred within 4 minutes, more preferred within 3 minutes, more preferred within 2 minutes, most preferred within 1 minute after oral administration of the dual drug delivery device. It is more preferred that at least 70% of the testosterone is released within 5 minutes, more preferred within 4 minutes, more preferred within 3 minutes, more preferred within 2 minutes, most preferred within 1 minute after oral administration of the dual drug delivery device.

As said, the present invention is an improvement over WO 2012/158030. Hence the provided time controlled, immediate release drug delivery system of step i) comprises a core comprising cellulose, a filler selected from an organic and/or an inorganic salt, and a first active ingredient, being an active ingredient for treatment male or female sexual dysfunction, decreased/absence of sexual desire, sexual arousal problems, or erectile dysfunction, and preferably for the treatment of Hypoactive Sexual Desire Disorder in men, or Female Sexual Interest / Arousal Disorder, and a first coating surrounding said core, said first coating comprising a hydrophobic polymer and a hydrophilic substance.

Preferably said core comprises
said first active ingredient in a relative amount of preferably between 0.1 and 60% (w/w; based on the total weight of the core), more preferred between 0.1 and 30% (w/w; based on the total weight of the core), more preferred between 1 and 25% (w/w based on the total weight of the core),
cellulose in a relative amount of preferably between 10 and 60% (w/w based on the total weight of the core), more preferred between 10 and 50 % (w/w based on the total weight of the core (w/w based on the total weight of the core), and
a filler selected from an organic and/or inorganic salt in a relative amount of preferably between 10 and 70% (w/w based on the total weight of the core), more preferred in an amount of between 10 and 60% (w/w based on the total weight of the core).

Preferably said filler is an inorganic salt; preferably, said cellulose is microcrystalline cellulose.

As mentioned above, the first active ingredient is preferably Sildenafil, and especially Sildenafil citrate, or Buspirone, and especially Buspirone HCl. When Buspirone is used, it was found that with the filler calcium sulphate when used instead of calcium phosphate, identified as preferred in WO 2012/158030, even better and more stable results were obtained.

In accordance with the present invention, the prepared system preferably is a tablet.

The core of the system of the present invention may additionally comprise a water-insoluble gel-forming disintegrant comprising cross-linked sodium carboxy methylcellulose, sodium starch glycolate and/or cross-linked polyvinylpyrrolidone.

Throughout this specification, the term "comprising" and its grammatical equivalents indicate that the components listed are present and that other components may be present or not.

The core is preferably pressed or compacted into a solid. A preferred core is a tablet. The term "tablet" encompasses a "capsule" and a "caplet". The preferred size of the core of a drug delivery system according to the invention ranges from a few millimeters to about one centimeter. Further excipients may include diluents, binders or granulating ingredients, a carbohydrate such as starch, a starch derivative such as starch acetate and/or maltodextrin, a polyol such as xylitol, sorbitol and/or mannitol, lactose such as α-lactose monohydrate, anhydrous α-lactose, anhydrous β-lactose, spray-dried lactose, and/or agglomerated lactose, a sugar such as dextrose, maltose, dextrate and/or inulin, or combinations thereof, glidants (flow aids) and lubricants to ensure efficient tabletting, and sweeteners or flavours to enhance taste.

Said first active ingredient can be a single active ingredient, or a mixture of two or more active ingredients. It is preferred that each of the active ingredients in a mixture of active ingredients is present in a relative amount of between 0.1 and 30% (w/w), more preferred between 1 and 25% (w/w).

A preferred time controlled, immediate release drug delivery system according to the invention comprises an immediate release formulation comprising a compressed core containing one or more active ingredients surrounded with a coating, wherein release of the active ingredient from the core is caused by rupture of the coating after a pre-defined lag-time. Preferably, the core disintegrates immediately after rupture or dissolution of the coating.

The term cellulose comprises powdered cellulose, agglomerated cellulose, microcrystalline cellulose and/or combinations thereof. The term cellulose includes purified cellulose, methylcellulose, hydroxypropyl methylcellulose, and carboxy methyl cellulose. Powdered cellulose is composed mainly of cellulose obtained by decomposing pulp. Microcrystalline cellulose comprises a special grade of alpha cellulose.

A preferred cellulose is microcrystalline cellulose. A preferred microcrystalline cellulose has a nominal particle size of between 30 and 250 µm, preferably of between 50 and 180 µm. A further preferred microcrystalline cellulose comprises a moisture of between 0.1 and 7.5 %, more preferred between 1 and 5.0 %. A preferred microcrystalline cellulose is selected from microcrystalline cellulose with a nominal particle size of 50 µm and a moisture of 3.0 to 5.0 % such as, for example, Avicel PH 101; a microcrystalline cellulose with a nominal particle size of 100 µm and a moisture of 3.0 to 5.0 % such as, for example, Avicel PH 102; and a microcrystalline cellulose with a nominal particle size of 180 µm and a moisture less than 1.5 % such as, for example, Avicel PH 200. The amount of said microcrystalline cellulose is preferably more than 10 % (w/w; based on the total weight of the core), more preferred more than 20 % (w/w), more preferred more than 30 %, most preferred more than about 35%. It is further preferred that the amount of microcrystalline cellulose is less than 60%, more preferred less than 55%, more preferred less than 50% (w/w, based on the total weight of the core).

A preferred core according to the invention comprises a filler. Said filler is preferably present in an amount of between 10 and 70% (w/w; based on the total weight of the core), more preferred between 20 % and 60% (w/w), more preferred between 30 % and 50% (w/w), such as, for example, 35% (w/w). Said filler is selected from the group of an organic salt and an inorganic salt. An organic salt is preferably selected from calcium citrate, magnesium citrate, calcium lactate, sodium lactate, magnesium lactate, calcium fumarate and magnesium fumarate. A most preferred filler is an inorganic salt. An inorganic salt according to the invention is preferably selected from calcium sulphate dihydrate, calcium silicate, silicium phosphate, calcium carbonate, anhydrous dibasic calcium phosphate, dibasic calcium phosphate monohydrate, tribasic calcium phosphate, sodium phosphate, sodium chloride, potassium phosphate, potassium sulphate, potassium chloride, sodium carbonate, magnesium carbonate, and magnesium oxide. The total amount of a soluble filler such as sodium lactate and sodium chloride is preferably below 50% (w/w; based on the total weight of the core). The selection of a filler is further determined by the intrinsic stability of the active ingredient in the core in combination with a filler or combination of fillers, as is known to the person skilled in the art. The core may further comprise a lubricant such as magnesium stearate, talc and the like. A preferred core comprises anhydrous dibasic calcium phosphate or calcium sulphate dihydrate and magnesium stearate. The amount of said anhydrous dibasic calcium phosphate is preferably more than 10 % (w/w; based on the total weight of the core), more preferred more than 20 % (w/w), more preferred more than 25 %, most preferred more than about 30%. It is further preferred that the amount of anhydrous dibasic calcium phosphate is less than 70%, more preferred less than 60%, more preferred less than 50%, more preferred less than 45% (w/w, based on the total weight of the core). The amount of magnesium stearate is preferably between 0.1 % (w/w; based on the total weight of the core) and 10 % (w/w), more preferred between 0.5 and 5 % (w/w). Another preferred core comprises calcium sulphate dihydrate and magnesium stearate. The amount of said calcium sulphate dihydrate is preferably more than 10 % (w/w; based on the total weight of the core), more preferred more than 20 % (w/w), more preferred more than 30 %, most preferred more than about 40%. It is further preferred that the amount of calcium sulphate dihydrate is less than 70%, more preferred less than 60%, more preferred less than 55%, more preferred less than 50% (w/w, based on the total weight of the core). The amount of magnesium stearate is preferably between 0.1 % (w/w; based on the total weight of the core) and 10 % (w/w), more preferred between 0.5 and 5 % (w/w).

The core additionally may comprise one or more disintegrants that, as a pure material, form a gel upon exposure to an aqueous liquid. A preferred disintegrant comprises one of more of a water-insoluble, gel-forming disintegrant. When present, said disintegrant such as a water-insoluble, gel-forming disintegrant is preferably present in a relative amount of between 0.5 and 20 % (w/w). Disintegrants are substances or a mixture of substances that facilitate the breakup or disintegration of a tablet. Break up of a tablet results in smaller particles of which the ingredients, including the first active ingredient, are more rapidly available for uptake, compared to a whole tablet. Drug dissolution can be improved significantly with the addition of disintegrating ingredients into the formulation. Preferred disintegrants induce disintegration of a tablet by wicking, deformation, and/or the induction of electric repulsive forces between particles.

A preferred disintegrant according to the invention is selected from sodium starch glycolate (Primojel®), cross-linked sodium carboxymethyl cellulose, for example ACDISOL®, cross-linked polyvinylpyrrolidone (Crospovidone) and low-substituted hydroxypropylcellulose (L-HPC) having a hydroxypropoxyl content in the range of 5.0 to 16.0% by weight and an apparent average degree of polymerization in the range of 350 to 700. Said L-HPC preferably has a low particle size, preferably below 10 microns average particle size, more preferred below 5 micron, such as, for example, LH41. Said water-insoluble, gel-forming disintegrant is preferably present in a relative amount of between 0.0 and 6 % (w/w). The amount of said water-insoluble gel-forming disintegrant is preferably less than 6 % (w/w; based on the total weight of the core), more preferred less than 5 % (w/w), most preferred less than 4 %.

A preferred composition of a core according to the invention comprises a first active ingredient, a microcrystalline cellulose, for example PHARMACEL^{(R)} pH102 or PHARMACEL^{(R)} pH200, anhydrous dicalcium phosphate, a crosslinked sodium carboxy methylcellulose, for example croscarmellose, and magnesium stearate. Microcrystalline cellulose and crosslinked sodium carboxy methylcellulose are preferably present in a ratio of between about 6:1 (w/w) to 14:1 (w/w), preferably between 7.5 (w/w) and 12.5 (w/w). Preferred ratios are about 10 : 1 (w/w) and about 8:1 (w/w). An effect of such ratio is that the core, while gel-forming, does not substantially swell prior to disintegration. A preferred ratio of anhydrous dibasic calcium phosphate and microcrystalline cellulose is between 3 : 1 (w/w) and 1 : 3 (w/w), more preferred between 2 : 1 (w/w) and 1 : 2 (w/w), most preferred in about 1 : 1 (w/w).

Another preferred composition of a core according to the invention comprises a first active ingredient, a microcrystalline cellulose, for example PHARMACEL^{(R)} pH102 or PHARMACEL^{(R)} pH200, calcium sulphate dihydrate, a crosslinked sodium carboxy methylcellulose, for example croscarmellose, and magnesium stearate. Microcrystalline cellulose and crosslinked sodium carboxy methylcellulose are preferably present in a ratio of between about 6:1 (w/w) to 14:1 (w/w), preferably between 7 (w/w) and 13 (w/w). Preferred ratios are about 12 : 1 (w/w) and about 10 : 1 (w/w). An effect of such ratio is that the core, while gel-forming, does not substantially swell prior to disintegration. A preferred ratio of calcium sulphate dehydrate and microcrystalline cellulose is between 3 : 1 (w/w) and 1 : 3 (w/w), more preferred between 2 : 1 (w/w) and 1 : 2 (w/w), most preferred in about 1 : 1 (w/w). The total weight of a core according to the invention is preferably between 50 and 500 milligram, more preferred between 200 and 400 milligram, more preferred between 250 and 350 milligram, such as about 300 milligram.

A core according to the invention is surrounded by a first coating, said first coating comprising an outer surface, said first coating further comprising a hydrophobic polymer and a (water-soluble and/or water-insoluble) hydrophilic substance. The first coating preferably does not comprise a drug. When present, a plasticizer such as, for example, dibutyl phthalate, triethyl citrate, acetyl triethyl citrate, dibutyl sebacate, diethyl phthalate, triacetin and/or tributyl citrate is preferably present in an amount of at most 0.5% (w/w; based on the total weight of the time controlled, immediate release drug delivery system). The first coating preferably does not comprise a plasticizer.

The first coating is preferably sprayed, for example with a nozzle, onto the core. For this, the hydrophobic polymer and water-soluble and/or water-insoluble hydrophilic substance are suspended or dissolved, for example and preferably, in a mixture of water and ethanol, and sprayed onto the core until a predetermined amount of testosterone in the first coating is obtained. The amount of the first coating is preferably between about 0.5 and 30 % (w/w) of the total weight of the time controlled, immediate release drug delivery system, more preferred between about 1 and 20 % (w/w).

A hydrophobic coating polymer according to the invention is preferably selected from water-insoluble coating materials such as cellulose derivates and polymethacrylates that are generated, for example, by copolymerization of methacrylate monomers with hydrophobic groups. Preferred polymethacrylate hydrophobic polymers are EUDRAGIT^{®} RL, EUDRAGIT^{®} RS, EUDRAGIT^{®} NE, and EUDRAGIT^{®} S.

Preferred cellulose derivates are selected from ethylcellulose and derivatives thereof. A most preferred hydrophobic polymer of the first coating of a drug delivery system according to the invention comprises ethylcellulose. Ethylcellulose forms a mechanically weak hydrophobic film that ruptures easily. The core contains a drug in combination with a water-insoluble, gel-forming disintegrant that disintegrates upon contact with an aqueous medium. The formation of pores in the hydrophobic film, and the influx of water into the core, causes the rupture of the ethylcellulose coating. When the coating is ruptured, the core disintegrates within minutes followed by the release of the drug. A preferred ethylcellulose is ETHOCEL^{®}.

A hydrophilic substance according to the invention preferably is a water-insoluble hydrophylic substance, preferably a water-insoluble hydrophylic polymer. It is further preferred that said first coating comprises pores prior to exposure to an aqueous liquid. The pores function as channels that interconnect the core with the outer surface of the inner coat for controlling the entry of aqueous liquid into the core. Said pores are present, for example, when the water-insoluble hydrophilic substance is or comprises a water-insoluble hydrophylic polymer, preferably cellulose. Preferred celluloses are cellulose derivatives such as, for example, hydroxypropylcellulose, crosslinked hydroxyethylcellulose, crosslinked hydroxypropylmethylcellulose and microcrystalline cellulose. Cellulose formed channels that connect the drug-containing core with the outside of the tablet. The cellulose thereby controls the rate at which water is being transported through the channels into the core. When sufficient water reaches the core, the core looses its structural integrity. The core will disintegrate, followed by rupture of the coating and release of the drug. A preferred cellulose is a microcrystalline cellulose with a nominal particle size of between 20 and 200 micron and a moisture of less than 5 %. A preferred microcrystalline cellulose comprises a microcrystalline cellulose with a nominal particle size of about 150 micron and a moisture of 3.0 to 5.0 % such as, for example, Avicel^{®} PH-102 SCG; a microcrystalline cellulose with a nominal particle size of about 100 micron and a moisture less than 5.0 % such as, for example Avicel^{®} HFE-102; a microcrystalline cellulose with a nominal particle size of about 20 micron and a moisture less than 5.0 % such as, for example, Avicel^{®} PH-105. Further preferred water insoluble hydrophilic substances include dicalcium phosphate.

An advantage of using smaller particles of less than 50 micron, e.g. Avicel^{®} PH-105, is that the coating suspension has better flow properties, which improves the overall film coating process. A preferred first coating comprises Ethocel^{®} and Avicel PH-105 as a water-insoluble hydrophylic substance. Preferred mass ratios of a hydrophobic coating polymer such as Ethocel^{®} and a water-insoluble hydrophilic substance such as Avicel are between 1: 5 and 5:1, more preferred between 1: 4 and 3:1, more preferred between 1: 3 and 2:1, most preferred about 1:2.

In another embodiment, a hydrophilic substance according to the invention preferably is a water-soluble hydrophilic substance. This coating preferably does not comprise pores or only a few pores prior to exposure to an aqueous liquid. It is preferred that the water-soluble hydrophilic substance forms pores in the hydrophobic polymer upon exposure to an aqueous liquid. A preferred water-soluble hydrophilic substance comprises lactose, mannitol and/or sodium chloride. A preferred lactose is PHARMATOSE®.

A preferred first coating comprises Ethocel^{®} and lactose as a water-soluble hydrophylic substance. Preferred mass ratios of a hydrophobic coating polymer such as Ethocel^{®} and a water-soluble hydrophilic substance such as lactose are between 1: 5 and 5:1, more preferred between 1: 3 and 3:1, more preferred between 1: 2 and 2:1, most preferred about 1:1.

The relative amount of a first coating is preferably between 4 and 20 % (w/w; based on the total weight of the drug delivery system), more preferred between 8 and 15 % (w/w), most preferred about 12% (w/w). Therefore, a preferred first coating has a weight of between 10 and 75 milligram, more preferred between 25 and 50 milligram, most preferred about 40 milligram.

A time controlled, immediate release drug delivery system according to the invention allows control of the release of a first active ingredient after hydration of the drug delivery system. Said time controlled, immediate release is essentially independent of pH. The timing is controlled in part by the thickness of the first coating, which is preferably sprayed onto the core. The variation in the amount of a first coating between tablets is preferably not more than 10 % (between 90 % and 110 %), based on the total weight of the first coating. More preferred, the variation in the amount of a first coating is not more than 5 % (between 95 % and 105 %), based on the total weight of the first coating. Factors (process conditions) that may influence the intra- en inter-tablet uniformity of the first coating include, for example, pan speed, spray rate, spray pattern, nozzle type, viscosity, drying temperature, air flow rate and coating time, as is known to the skilled person. When required, a temperature controlled curing step, for example heat treatment at 60-80 °C for 1-3 hours, is applied to the first coating after application, preferably spraying, of the first coating.

In addition, the amounts of the water-soluble and/or water-insoluble hydrophilic substance in the first coating, and the identity of the water-soluble and/or water-insoluble hydrophilic substance, further provide means to modulate the timing of release of a first active ingredient. For example, a tablet comprising a pressed core and a first coating with an average thickness of about 35 micrometer, the coating comprising Ethocel 20 and lactose in a 3: 2 ratio, provides release of the first active ingredient at about 36 minutes after hydration of the tablet, while the same composition of a tablet with a first coating with an average thickness of about 50 micrometer, provides release of the first active ingredient at about 84 minutes after hydration of the tablet. A tablet comprising a pressed core and a first coating with an average thickness of about 90 micrometer, the coating comprising Ethocel 20 and Avicel PH102 in a 3: 2 ratio, provides release of the first active ingredient at about 105 minutes after hydration of the tablet. The skilled person is able to generate a time controlled, immediate release drug delivery system according to the invention, based on the teaching and the examples provided in this application.

The total weight of a drug delivery device according to the invention is preferably at least 50 milligram, more preferred at least 150 milligram, and preferably is between 50 and 500 milligram, more preferred between 150 and 400 milligram, more preferred between 300 and 400 milligram, such as about 301.5 milligram, 325 milligram, or about 340 milligram.

The term "time controlled" drug delivery system refers to a drug delivery system that provides release of a first active ingredient after a predetermined period of time, for example 2.5 hours, whereby the release is independent of pH. The predetermined period of time is set and not dependent on the pH history in the gastro-intestinal tract.

The term "immediate release" drug delivery system refers to a drug delivery system that provides release of a substantial amount of a first active ingredient within a predefined period of time. An immediate release drug delivery system, for example, provides the release of more than 60% of a first active ingredient, more preferred more than 70%, more preferred more than 80%, within 30 minutes after rupture of the coating, more preferred within 20 minutes, more preferred within 8 minutes after rupture of the coating. Methods and means to determine the amount of a first active ingredient that is released from a drug delivery system, and the time frame within which the ingredient is released, such as for example compendial dissolution methods, are known to the skilled person such as, for example, United States Pharmacopoeia (USP) dissolution tests based on Apparatus 2 (the paddle method) and Apparatus 3 (the reciprocating cylinder).

The immediate release of a first active ingredient is thought to be caused by moisture induced stress relaxation. The driving force for this stress relaxation is the amount of stored energy within the core as surrounded by the polymer coating (Van der Voort Maarschalk et al., 1997. Int J Pharmaceutics 151: 27-34; Van der Voort Maarschalk et al., 1997. Pharm Res 14: 415-419; Steendam et al., 2001. J Control Rel 70: 71-82; Laity and Cameron, 2010. Eur J Pharm Biopharm 75: 263-276). Stress relaxation mediates the breakage of a coated core according to the invention in a nonlinear fashion. Hydration of the core and the hydrophilic substance in the first coating mediates stress relaxation such that an immediate burst of the coating after a predetermined period of time is obtained. It was found that the presence of more than 6 % (w/w) of a water-insoluble, gel-forming disintegrant interferes with the immediate release of a first active ingredient and leads to more sustained release properties.

The spray coating comprising testosterone or a functional analogue thereof preferably comprises a carrier selected from hydroxypropyl-beta-cyclodextrin, poly-beta-cyclodextrin, gamma-cyclodextrin and polyvinylpyrrolidone. A preferred polyvinylpyrolidone is low molecular weight polyvinylpyrrolidone with a molecular weight of maximal 80000. A suitable polyvinylpyrrolidone is preferably selected from K10, K15, K25, K30, and K50. A most preferred carrier is hydroxypropyl-beta-cyclodextrine. The presence of a poorly soluble steroid such as testosterone and a carrier such as a cyclodextrin provides rapid and efficient delivery of the testosterone to the mucous membrane, from which the steroid is than rapidly absorbed into the circulation. The amount of said carrier is preferably between 0.5 and 70 % (w/w), based on the total weight of the spray coating, more preferred between 2 and 60 % (w/w), more preferred between 5 and 50 % (w/w).

The spray coating preferably comprises a flavouring compound and one or more excipients, such as, for example, a colouring agent. Said flavouring compound may be any natural, artificial or synthetic compound or mixture of compounds that is pharmaceutically acceptable. An illustrative list of flavours for pharmaceutical applications includes cyclic alcohols, volatile oils, synthetic flavour oils, flavouring aromatics, oils, liquids, oleoresins and extracts derived from plants, leaves, flowers, fruits, stems, roots, and combinations thereof. Non-limiting examples of cyclic alcohols include menthol, isomenthol, neomenthol and neoisomenthol. Non-limiting examples of flavour oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, cassia oil, and combinations thereof. Suitable flavours also include, for example, artificial, natural and synthetic fruit flavours such as citrus oils (e.g., lemon, orange, lime, and grapefruit), fruit essences (e.g., lemon, orange, lime, grapefruit, apple, pear, peach, grape, strawberry, raspberry, cherry, plum, pineapple, apricot or other fruit flavours). Other useful artificial, natural and synthetic flavours include sugars, polyols such as sugar alcohols, artificial sweeteners such as aspartame, stevia, sucralose, neotame, acesulfame potassium, and saccharin, chocolate, coffee, vanilla, honey powders, and combinations thereof. Other useful flavours include aldehydes and esters, such as benzaldehyde (cherry, almond), citral (lemon, lime), neral (lemon, lime), decanal (orange, lemon), aldehyde C-8 (citrus fruits), aldehyde C- 9 (citrus fruits), aldehyde C-12 (citrus fruits), tolyl aldehyde (cherry, almond), 2,6- dimethyloctanal (green fruit), 2-dodenal (citrus mandarin), and combinations thereof. A preferred flavouring compound is a cyclic alcohol such as, for example, menthol, isomenthol, neomenthol and neoisomenthol, preferably combined with an artificial sweetener such as aspartame. The amount of a flavouring compound is preferably between 0. 1 and 60 % (w/w), based on the total weight of the spray coating, more preferred between 1 and 40 % (w/w).

The presence of a flavouring compound in the spray coating of a dual drug delivery device according to the invention may mask a bitter or objectional-tasting drug or excipient.

It is preferred however that the flavouring compound in the spray coating of a dual drug delivery device according to the invention rapidly disappears from the oral cavity. Sensing of the particular flavour in the oral cavity indicates to the user that the spray coating has not completely dissolved and that the time controlled, immediate release drug delivery system which is encompassed within the spray coating is to be held in the mouth. During use, the testosterone is co-delivered with the flavouring compound from the spray coating. A subject can easily recognize that the device is delivering the testosterone due to the presence of the flavour (taste). Eventually, the entire dose of testosterone is delivered. At this point, the device also stops delivering the flavour. The disappearance of the flavour (taste) indicates that the time controlled, immediate release drug delivery system may be swallowed.

The skilled person will understand that a flavouring compound may be present in the first coating, instead of in the spray coating. In that case, the appearance of the flavour (taste) indicates that the time controlled, immediate release drug delivery system may be swallowed. The skilled person will further understand that a first flavouring compound may be present in the spray coating, while a second flavouring compound is present in the first coating. Upon disappearance of the first flavour (taste), and tasting of the second flavour (taste), the subject knows that the device has delivered the entire dose of the testosterone.

It is further preferred that the roughness of the outer surface of the spray coating differs from the roughness of the outer surface of the first coating in a device according to the invention. A subject can be instructed to swallow the time controlled, immediate release drug delivery system when a difference in roughness becomes evident. This provides sufficient retention time of a device according to the invention in the mouth so that the testosterone is sufficiently released and absorbed.

The present invention will now be further illustrated while referring to the following, non-limiting working examples. If reference is made to percentages for a particular compound, these are weight percentages, drawn to the weight of the composition or layer in which said compound is present, unless otherwise specified. The materials used in the working examples are obtained from the same manufacturers as described in WO 2012/158030.

### EXAMPLE 1

4.6 kg sildenafil citrate, 6.7 kg dicalcium phosphate, 6.7 kg microcrystalline cellulose and 0.8 kg croscarmellose sodium were passed through a 600 micrometer sieve in a blending container and blended until a uniform mass was obtained. Then, 0.8 kg magnesium stearate was passed through a 600 micrometer sieve and added to the blend. The blend was lubricated by tumbling the blending container for 5 minutes. The blend was transferred to a tableting machine and compressed to tablets having a core weight of 300 mg, containing about 70 mg sildenafil citrate equivalent to 50 mg sildenafil per tablet.

In 14.4 liters ethanol (96%, denaturated), 432 g ethylcellulose (20 mPa.s) and 864 g microcrystalline cellulose (Avicel PH105) were dispersed. The 7.2 kg sildenafil core tablets were loaded in a perforated drum film coater. The ethylcellulose and microcrystalline cellulose dispersion was subsequently sprayed on the sildenafil tablets and the solvent was removed by heating, while maintaining the exhaust temperature at about 40°C.

The coated tablets were gradually cooled.

During drug product stability testing, it was determined that the properties of the polymer barrier coating surrounding the sildenafil core changed upon storage. This resulted in a delayed dissolution profile of sildenafil from the original target of 2 to 3 hours to a time greater than 3 hours.

This effect was not observed in the tablets prepared in the working examples of WO 2012/158030.

It has been determined that the delayed release of sildenafil was attributable to an ageing, or curing, phenomenon of the ethylcellulose-containing polymer coating. More specifically, in an effort to understand the cause of the delayed sildenafil core tablet rupture time on stability, it became apparent that the nature of the ethylcellulose polymer coating changed during storage, which, in turn, changed its swelling properties over time. This change was due to a curing effect - rearrangement of the polymer strains reduces the density of the coating, thereby reducing the speed of water uptake.

It was realized that in WO 2012/158030 the coat layers were applied on small-scale laboratory equipment and the drying temperature was uncontrolled (estimated to be between 60°C and 80°C).

Therefore, the effects of Additional Heat Treatment on Initial Rupture Time were checked.

Specifically, core tablets containing 50 mg sildenafil of the type made in Example 1 were coated with approximately 34 mg per tablet of barrier coating comprised of ethylcellulose and microcrystalline cellulose. After spraying of the barrier coating, samples were taken for analysis of core rupture time. The tablets were further processed in the film-coating equipment by applying heat (supply air 70°C to 80°C, resulting in exhaust air > 60°C) for 3 hours. Samples were taken after 1, 2 and 3 hours of additional heat treatment to investigate the polymer coat curing effect.

The data, presented in Table 1 herein-below, demonstrated that this additional heating step shifted the average rupture time of the sildenafil core tablets from 90 minutes to an average of about 120 minutes. No clear differences in rupture time were observed among heat treatments of 1, 2 or 3 hours.

**Table 1: Effect of Additional Heat Treatment on Sildenafil Core Rupture Time**

| | | **Additional Drying Time** | | |
|---|---|---|---|---|
| | | **With Supply Air at 70°C to 80°C** | | |
| **Parameter** | **End of Spraying** | **1 hour** | **2 hours** | **3 hours** |
| **Weight of 20 tablets** (g) | 6.6461 | 6.6040 | 6.6353 | 6.5968 |
| (Initial testing at time zero) | | | | |
| **Rupture time** (minutes) | | | | |
| (n = 6) | 105 | 110 | 121 | 117 |
| | 95 | 119 | 133 | 105 |
| | 81 | 129 | 106 | 124 |
| | 86 | 119 | 129 | 127 |
| | 83 | 118 | 127 | 129 |
| | 91 | 113 | 110 | 137 |
| Average | 90.2 | 118.0 | 121.0 | 123.2 |
| Minimum | 81 | 110 | 106 | 105 |
| Maximum | 105 | 129 | 133 | 137 |
| Standard deviation | 8.91 | 6.51 | 10.86 | 11.03 |

### Stability of Experimental Batch With Additional Heat Treatment

Samples from the batch of Example 1 with the additional heat treatment for 1 hour and for 3 hours were stored up to 32 days under the conditions listed below. Stability data on the batch of Example 1 (*i.e*., coating without the additional heat treatment) served as the control for this experiment.
- Samples heat-treated for 1 additional hour were stored in the open at 40°C/75% RH for 32 days.
- Samples heat-treated for 3 additional hours were stored as follows:
- in the open at 40°C/75% RH;
- packed in high-density polyethylene (HDPE) bottles and stored at 40°C/75% RH; and
- packed in HDPE bottles and stored at 4°C to 8°C.

The results for average rupture time are presented in Table 2.

**Table 2: Average Rupture Time After Storage of Experimental Batch 2112/004 Rupture Time (minutes)**

| | **Additional Drying Time With Supply Air at 70°C to 80°C** | | | |
|---|---|---|---|---|
| | **1 hour** | **3 hours** | **3 hours** | **3 hours** |
| **Storage Conditions** | Open 40°C/75% RH | Open 40°C/75% RH | HDPE Bottle 40°C/75% RH | HDPE Bottle 4°C-8°C |
| **Storage Time (days)** | | | | |
| 0 | 118 | 123 | 123 | 123 |
| 7 | 121 | 124 | nd | nd |
| 20 | 130 | 115 | 115 | 124 |
| 32 | 117 | 114 | 125 | 122 |

| | | | | |
|---|---|---|---|---|
| Note: nd = not determined | | | | |

The results indicated that, despite the expected intrinsic variability in the rupture time of the tablets, curing was obtained by applying an additional heat treatment to the ethylcellulose polymer coat. After curing, no further aging which might influence the swelling and water-penetration properties of the polymer coating was observed.

When these tablets prepared according to this present example 1 were cured in a perforated drum film coater for 60 min at a tablet bed temperature of 67 - 69°C, the undesired product stability effects did not occur.

### EXAMPLE 2

0.3 kg buspirone HCl, 4.1 kg calcium sulphate dihydrate, 4.1 kg microcrystalline cellulose and 0.36 kg croscarmellose sodium were passed through a 600 micrometer sieve in a blending container and blended until a uniform mass was obtained. Then, 0.12 kg magnesium stearate was passed through a 600 micrometer sieve and added to the blend. The blend was lubricated by tumbling the blending container for 5 minutes. The blend was transferred to a tableting machine and compressed to tablets having a core weight of 300 mg, containing about 10 mg Buspirone hydrochloride per tablet. In 14.4 liters ethanol (96%, denaturated), 432 g ethylcellulose (20 mPa.s) and 864 g microcrystalline cellulose (Avicel PH105) were dispersed. The 7.2 kg buspirone core tablets were loaded in a perforated drum film coater. The ethylcellulose and microcrystalline cellulose dispersion was subsequently sprayed on the Buspirone tablets and the solvent was removed by heating, while maintaining the exhaust temperature at about 40°C.

The coated tablets were gradually cooled.

Afterwards the tablets were cured in a perforated drum film coater for 60 min at a tablet bed temperature of 67 - 69°C.

### EXAMPLE 3

In 10.1 liters of a 70:30 mixture of ethanol (96%, denatured):purified water, 18 g testosterone, 48 g of hypromellose (5 mPa.s), 96 g hydroxypropyl β-cyclodextrin, 36 g aspartame and 22 g menthol were added. More specifically, the cyclodextrin was dispersed in an aliquot of purified water; all ethanol was added; the testosterone and menthol were dissolved upon stirring. Hypromellose was added to the remainder of the water and stirred until dispersed. Both liquid phases were combined. Subsequently, the aspartame was added ant stirred until dissolved. A visibly clear solution was obtained.

7.2 kg of barrier coated tablets as prepared in Example 2 were added in a 50 cm coating pan and placed in a coating machine equipped with a Schlick model 930/7-1 S35 (1.2 mm nozzle). Using a pan speed of 19 rpm, approximately 15 cm distance from spray gun to tablets and a 300 m³/hr supply air volume, atomizing air pressure of 0.7 bar and 1.0 bar pattern air pressure and an exhaust air temperature of 40-42°C the tablets were coated at a target spray rate of 18-22 g/min.

In this way, it was found that on an industrial scale tablets were obtained wherein testosterone was present in the spray coating layer of each individual tablet within 3.6% of the average amount. Table 3 lists the individual data, the mean, the (relative) standard deviation and the acceptance value (AV) as the USP and Ph. Eur quality standard in content uniformity testing.

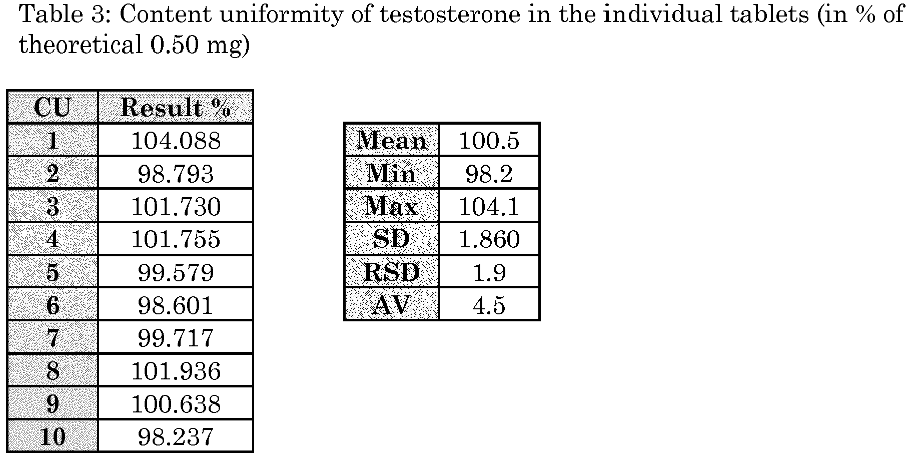

## Claims

1. A process for preparing dual drug delivery devices for oral administration of a second and first active ingredient to a subject in need thereof, said process comprising the steps of
(i) providing a time controlled, immediate release drug delivery system comprising a core comprising cellulose, a filler selected from an organic and/or an inorganic salt, and the first active ingredient, being an active ingredient for treatment male or female sexual dysfunction, decreased/absence of sexual desire, sexual arousal problems, or erectile dysfunction, and preferably for the treatment of Hypoactive Sexual Desire Disorder in men, or Female Sexual Interest / Arousal Disorder, and a first coating surrounding said core, said first coating comprising a hydrophobic polymer and a hydrophilic substance; and
(ii) spray coating said time controlled, immediate drug delivery systems with testosterone or a functional analogue or derivative thereof in amounts within 10% and preferably 7,5%, more preferably within 5%, most preferably within 4% of the desired pharmaceutical amounts.

2. The process according to claim 1, wherein testosterone or the functional analogue thereof is spray coated using a solution comprising a film forming polymer and a solvent system based on ethanol and water.

3. The process according to claim 2, wherein the film forming polymer is hydroxypropylmethylcellulose.

4. The process according to claim 2 or 3, wherein the weight ratio ethanol/water is 6/4 - 8/2.

5. The process according to any one of the preceding claims, wherein testosterone or the functional analogue thereof is spray coated using a solution further comprising a cyclodextrin, or a derivative or polymer thereof.

6. The process according to any one of the preceding claims, wherein testosterone or the functional analogue thereof is spray coated using a solution further comprises a flavouring compound and/or a sweetener.

7. The process according to any one of the preceding claims, wherein the first coating of step (i) is also applied using spray coating step.

8. The process according to any one of the preceding claims, wherein the spray coating step ii) and/or in the embodiment of claim 7, the spray coating step in step (i) inclusive of its inherent drying step is carried out at temperatures up to maximally 55°C, preferably up to maximally 50°C, and more preferably at temperatures in the range of 35°C to 45°C.

9. The process according to any one of the preceding claims, wherein the first coating around the core in step i) comprises ethylcellulose.

10. The process according to claim 9, wherein the first coating is applied at a temperature of 60°C or higher; or wherein the provided time controlled, immediate release drug delivery system of step i) is subjected to a curing step.

11. The process according to any one of the preceding claims, wherein the first active ingredient is Sildenafil or Buspirone.

12. The process according to any one of the preceding claims wherein the spray coating step or step is/are carried out in a perforated pan coater or fluid bed coater.

13. The dual drug delivery device obtainable by the process of any one of claims 1-12.
